# EUROPEAN PATENT APPLICATION

(11) **EP 2 324 847 A1**
(43) Date of publication of application: **25.05.2011**
(21) Application number: 09014652.3
(22) Date of filing: 24.11.2009
(51) Int. Cl.: A61K 38/57, C07K 14/81, C07K 19/00, C12N 15/62, A61P 17/02

(54) **Acceleration of wound healing by a tissue inhibitor of metalloproteinases (TIMP) linked to glycosylphosphatidylinositol (GPI)-anchors**

(71) Applicant: Nelson, Peter Jon, 80336 München (DE); Ludwig-Maximilians-Universität München, 80337 München (DE)
(72) Inventor: Nelson, Peter Jon, 81575 München (DE)
(74) Representative: Grund, Martin

(57) **Abstract**

The present invention relates to fusion constructs of glycosylphosphatidylinositol (GPI)-anchored tissue inhibitors of metalloproteinases (TIMPs) and their use for the treatment of cancer and in regenerative medicine. By this approach, the GPI-anchored TIMP proteins are incorporated into the surface membrane of cells. The fusion constructs of the present invention are effective agents in accelarating wound healing.

SEQUENCE LISTING

<110>  Nelson, Peter Jon
       LMU München

<120>  Acceleration of wound healing by a Tissue Inhibitor Of
       Metalloproteinases (TIMP) Linked To Glycosylphosphatidylinositol
       (GPI)-Anchors

<130>  109-005

<160>  3

<170>  PatentIn version 3.3

<210>  1
<211>  582
<212>  DNA
<213>  Artificial Sequence

<220>
<223>  Truncated human TIMP-1 fused to GPI sequence

<400>  1
gaattcatgg ccccctttga gcccctggct tctggcatcc tgttgttgct gtggctgata      60

gcccccagca gggcctgcac ctgtgtccca ccccacccac agacggcctt ctgcaattcc     120

gacctcgtca tcagggccaa gttcgtgggg acaccagaag tcaaccagac caccttatac     180

cagcgttatg agatcaagat gaccaagatg tataaagggt tccaagcctt aggggatgcc     240

gctgacatcc ggttcgtcta cacccccgcc atggagagtg tctgcggata cttccacagg     300

tcccacaacc gcagcgagga gtttctcatt gctggaaaac tgcaggatgg actcttgcac     360

atcactacct gcagttttgt ggctccctgg aacagcctga gcttagctca gcgccggggc     420

ttcaccaaga cctacactgt tggctgtgag gaatgcacag tgtctagaac aacctgtatc     480

ccaagcagcg gtcattcaag acacagatat gcacttatac ccataccatt agcagtaatt     540

acaacatgta ttgtgctgta tatgaatgta ttatgagtcg ac                        582


<210>  2
<211>  599
<212>  DNA
<213>  Artificial Sequence

<220>
<223>  Human TIMP-2 fused to GPI sequence

<400>  2
gaattcatgg caaccctaga gaggatccag tatgagatca agcagataaa gatgttcaaa      60

gggcctgaga aggatataga gtttatctac acggccccct cctcggcagt gtgtggggtc     120

tcgctggacg ttggaggaaa gaaggaatat ctcattgcag gaaaggccga gggggacggc     180

aagatgcaca tcaccctctg tgacttcatc gtgccctggg acaccctgag caccacccag     240

aagaagagcc tgaaccacag gtaccagatg ggctgcgagt gcaagatcac gcgctgcccc     300

atgatcccgt gctacatctc ctccccggac gagtgcctct ggatggactg ggtcacagag 360

aagaacatca acgggcacca ggccaagttc ttcgcctgca tcaagagaag tgacggctcc 420

tgtgcgtggt accgcggcgc ggcgcccccc aagcaggagt ttctcgacat cgaggaccca 480

tctagaacaa cctgtatccc aagcagcggt cattcaagac acagatatgc acttataccc 540

ataccattag cagtaattac aacatgtatt gtgctgtata tgaatgtatt atagtcgac 599

<210> 3
<211> 758
<212> DNA
<213> Artificial Sequence

<220>
<223> Mutated human TIMP-3 fused to GPI sequence

<400> 3
gaattcatga ccccttggct cgggctcatc gtgctcctgg gcagctggag cctgggggac 60

tggggcgccg aggcgtgcac atgctcgccc agccaccccc aggacgcctt ctgcaactcc 120

gacatcgtga tcgcggccgc ggtggtgggg gcgaagctgg tagcggaggg gcccttcggc 180

acgctggtct acaccatcgc gcagatggcg atgtaccgag gcttcaccaa gatgccccat 240

gtgcagtaca tccatacgga agcttccgag agtctctgtg gccttaagct ggaggtcaac 300

aagtaccagt acctgctgac aggtcgcgtc tatgatggca agatgtacac ggggctgtgc 360

aacttcgtgg agaggtggga ccagctcacc ctctcccagc gcaaggggct gaactatcgg 420

tatcacctgg gttgtaactg caagatcaag tcctgctact acctgccttg ctttgtgact 480

tccaagaacg agtgtctctg gaccgacatg ctctccaatt tcggttaccc tggctaccag 540

tccaaacact acgcctgcat ccggcagaag ggcggctact gcagctggta ccgaggatgg 600

gccccccggg ataaaagcat catcaatgcc acagacccct ctagaacaac ctgtatccca 660

agcagcggtc attcaagaca cagatatgca cttataccca taccattagc agtaattaca 720

acatgtattg tgctgtatat gaatgtatta tagtcgac 758

1

## Description

The present invention relates to use of TIMP fusion constructs for accelerating wound healing.

### BACKGROUND OF THE INVENTION

### The Role of TIMPs in Regenerative Medicine

One significant area in the field of regenerative medicine is concerned with the wound healing process. Wound healing relates to a natural restorative response to tissue injury and involves a complex cascade of cellular events that ultimately generates the resurfacing, reconstitution, and restoration of the tensile strength of injured tissue. This process generally engages the recruitment and proliferation of different cell types, an elaboration of the cellular matrix, and an increase in immune surveillance.

Wound healing is a complex physiological process involving the orchestration of a series of cellular and biochemical events that ultimately drive healing. Cutaneous wound repair can be divided into a series of overlapping phases including formation of the fibrin clot, the inflammatory response, granulation tissue formation incorporating re-epithelialisation and angiogenesis and finally, matrix formation and remodeling. Matrix metalloproteases (MMPs) are a family of proteases whose biology is interwoven through the wound healing process.

MMPs regulate inflammation, influence cell-cell interactions, degrade the extracellular matrix to facilitate the migration of cells, and remodel the new ECM. However, excessive MMP activity is thought to contribute to the development of chronic wounds. The activities of most MMPs are low in normal tissue but are rapidly upregulated during inflammation and tissue remodeling. The selective control of MMP activity may prove to be a valuable therapeutic approach to promote wound healing.

Wound healing proceeds in a timely, sequential manner and can be divided into four general phases: inflammation, granulation, re-epithelialization and tissue remodeling. Each phase of the wound healing process is regulated by special signal transduction pathways. During wound healing, an increase in the expression of growth factors and cytokines occurs; in particular, increases in the levels of TNF, IL-1, and IL-6, have been described. During the initial inflammation phase, which involves the effector proteins IL-1, TNF-α and CSF, both macrophages and neutrophils are recruited to form a fibrin clot. During the granulation phase, the fibroblasts proliferate, migrate to the wound, and secrete ECM. The effector proteins involved in this latter phase include MMPs, PDGF, FGF, EGF and VEGF. The third phase in wound healing, re-epithelialization, is characterized by the proliferation of keratinocytes, which migrate into the wound, and also by an increase in myofibroblasts, which are responsible for wound contraction. The result of this phase, which involves the effector proteins MMPs, KGF, TGF, GM-CSF, EGF and uPA and tPA, is the re-epithelialization of the wound surface, the dissection of eschar, and the formation of a barrier. Finally, during the tissue remodeling phase, fibroblasts produce a collagenous matrix leading to the formation of scar tissue, apoptosis of fibroblasts, and a switch from the activation to differentiation of the keratinocytes. Known effector proteins involved in this last phase of wound healing include TGF-b1, MMPs and TIMPs.

Thus, the effector cells responsible for most aspects of wound healing are the fibroblasts and the keratinocytes, and MMPs that play an important role in both the migration of fibroblasts (MMP-1, -2, -3 and -13) and keratinocytes (MMP-1, -2, -3, and -10) (Singer & Clark, 1999) in addition to scar formation. Each of the MMPs has a different substrate specificity within the ECM, and play an important role in ECM degradation and turnover. The MMP family includes, *inter alia,* collagenases (MMP-1, MMP-8, MMP-13, MMP-18), stromelysins (MMP-3, MMP-10, MMP-11), gelatinases (MMP-2, MMP-9), matrilysin (MMP-7), metalloelastase (MMP-12) and a series of membrane-bound matrix metalloproteinases (MT-MMPs). As the function of MMPs is to proteolytically break down the surrounding ECM, a balance between this protease activity and ECM deposition during wound healing, i.e. re-building of the injured tissue, needs to be optimally maintained. The control of MMP activity is modulated by the TIMP proteins, which are produced by most cells, and act to inhibit the MMPs in a 1:1 ratio.

Therefore, there exists a need to control or influence the physiological balance between protease activity and ECM deposition during the wound healing process.

We have previously reported that treatment of activated dermal fibroblast cells in vitro with TIMP-1-GPI effected diverse aspects of their biology. Surface treatment of these myofibroblasts lead to a reduction in their proliferation, migration and activation.

Surprisingly, it was found that TIMP constructs can not only suppress fibrosis, but can also accelerate treatment wound closure by activating the proliferation and migration of dermal keratinocytes. In a similar light, we have found that TIMP-1-GPI treatment can induce migration and proliferation of primary mesothelial cells suggesting that the agent may show efficacy in general wound repair.

In a further embodiment, the fusion constructs of the present invention provide an effective regenerative agent for the treatment of conditions defined by a disturbed balance between MMP protease activity and ECM deposition as, for example, in keloid scarring or chronic wounds, that are commonly associated with increased MMP levels. Additionally, the fusion constructs of the present invention provide an effective regenerative agent that can reduce, minimize or inhibit the formation of scars during the wound healing process.

### SUMMARY OF THE INVENTION

The invention is defined by the appended claims.

The invention relates to fusion construct, comprising an amino acid sequence of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor, for the preparation of a medicament for accelerating wound healing.

By accelerating wound healing is meant that the wound healing process occurs at least 1.1, 1.2, 1.3, 1.5, 1.6, 1.7, 1.8, 1.9, or 2.0 faster or within a range of any of the aforementioned values (for example, 1.1-2.0, 1.3-1.9, 1.4-1.8, 1.5-2.0, etc.) than the wound healing without any treatment or a treatment without any active substance.

The wound healing process can be measured by determining the change (shrinkage) of wound volume in the course of time. Example 1 discloses a method to measure the wound volume. Alternatively, an *in vitro* test like in Examples 2 or 5 can be used. Alternatively and preferentially, the proliferation and migration of primary human mesothelial cells or keratinocytes can be measured. The preferred time range for determining the change of wound volume in vivo or in vitro or the proliferation and migration of primary human mesothelial cells or keratinocytes can be 7, 6, 5, 4, or 3 days.

The subject to be treated can be an animal or a mammal and preferentially a human.

TIMP can be selected from the group consisting of TIMP-1, TIMP-2, TIMP-3, or TIMP-4.

The fusion construct can contain one or more GPI signal sequences to direct GPI-anchoring. The GPI-anchor can be derived from the lymphocyte function-associated antigen (LFA-3), or a portion thereof.

The fusion construct can be administered at a concentration of 0.005 to 0.1 µg/ml, 0.002 to 0.1 µg/ml, 0.002 to 0.015 µg/ml, 0.003 to 0.015 µg/ml, 0.004 to 0.015 µg/ml, 0.005 to 0.015 µg/ml, 0.005 to 0.010 µg/ml.

The pharmaceutical composition can be used together with a detergent, sealant or carrier substance.

The invention can also relate to a method of accelerating wound healing or measuring the influence of TIMP constructs on primary keratinocytes and mesothelial cells. The method may comprises administering a pharmaceutical composition comprising an amino acid sequence of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor. The method can be *an in vitro* method wherein said amino acid sequence is added to isolated cells. *The in vitro* method may be used to test the efficiency of TIMP constructs. The isolated cells can be primary keratinocytes and mesothelial cells. The method can be performed on animals, mammals or humans or isolated cells of the corresponding origin.

The in vitro test may be performed together with controls, that means no TIMP constructs are added to the control cells. The proliferation and/or mobilization of the cells that received the constructs can then be measured in comparison to the cells that did not receive the construct. The wound healing process can be measured by measuring the change (shrinkage) of wound volume in the course of time. Example 1 discloses a method to measure the wound volume. Alternatively, an *in vitro* test like in Examples 2 or 5 can be used. Alternatively and preferentially, the proliferation and migration of primary human mesothelial cells or keratinocytes can be measured. The preferred time range for measuring the change of wound volume in vivo or in vitro or the proliferation and migration of primary human mesothelial cells or keratinocytes can be 7, 6, 5, 4, or 3 days.

The subject to be treated can be an animal or a mammal and preferentially a human.

TIMP can be selected from the group consisting of TIMP-1, TIMP-2, TIMP-3, or TIMP-4.

The fusion construct can contain one or more GPI signal sequences to direct GPI-anchoring. The GPI-anchor can be derived from the lymphocyte function-associated antigen (LFA-3), or a portion thereof.

The fusion construct can be administered at a concentration of 0.005 to 0.1 µg/ml, 0.002 to 0.1 µg/ml, 0.002 to 0.015 µg/ml, 0.003 to 0.015 µg/ml, 0.004 to 0.015 µg/ml, 0.005 to 0.015 µg/ml, 0.005 to 0.010 µg/ml.

The pharmaceutical composition can be used together with a detergent, sealant or carrier substance. The detergent may be present in a concentration between 0.01 % to 0.05%. The detergent may be present in a concentration between 0.02 % to 0.03 %. The detergent may be present in a concentration 0.025 %. The detergent of the composition may be Triton X-100. The invention also relates to a pharmaceutical composition comprising 0.005 to 0.1 µg/ml, 0.002 to 0.1 µg/ml, 0.002 to 0.015 µg/ml, 0.003 to 0.015 µg/ml, 0.004 to 0.015 µg/ml, 0.005 to 0.015 µg/ml, 0.005 to 0.010 µg/ml of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor. TIMP may be suspended in water or PBS. For further possible formulation of TIMP we refer to EP 1764372.

The invention is characterized in that the above defined TIMP constructs effect the proliferation and mobilization of keratinocytes and/or mesothelial cells in wounds.

Disclosed is also a fusion construct (TIMP-GPI) comprising an amino acid sequence of a tissue inhibitor of metalloproteinases (TIMP) or a biologically active fragment thereof, wherein said TIMP or biologically active fragment thereof is linked to an amino acid sequence of a mucin domain followed by an amino acid sequence of a glycosylphosphatidylinositol (GPI)-anchor.

Disclosed is that the 3'-end of TIMP is fused directly to a GPI-linking sequence.

The TIMP as used in the present is preferably derived from a mammal; more preferred is a human (the four TIMPs are reviewed in Mannello F *et al.,* 2001). Examples of TIMP proteins that can be used in accordance with the present invention comprise TIMP-1, TIMP-2, TIMP-3, or TIMP-4 and their corresponding variants in other organisms such as mouse, rabbit, dog, cat, sheep, and cow.

The GPI-anchor as used in the present invention is preferably derived from the lymphocyte function-associated antigen (LFA-3), or a portion thereof, and includes a GPI-signal sequence that mediates membrane association.

The present invention also relates to a nucleic acid molecule, such as RNA or DNA, comprising a nucleic acid sequence that encodes for the GPI-anchored TIMP construct of the invention.

In a further aspect of the present invention, the nucleic acid molecule of the invention is contained in an expression plasmid, a vector or a host cell for expression of the nucleic acid molecule of the invention.

In a further aspect of the present invention, the TIMP-GPI fusion constructs of the invention are contained in a pharmaceutical composition or medicament

In a further embodiment, the present invention provides novel agents and methods for accelerating wound healing.

### DEFINITIONS

With the term "TIMP" as used herein is meant an endogenous tissue inhibitor of metalloproteinases, which is known to be involved in physiological/biological functions including the inhibition of active matrix metalloproteinases, regulation of pro MMP activation, cell growth, and the modulation of angiogenesis. The human "TIMP family" contains four members, TIMP-1, TIMP-2, TIMP-3, and TIMP-4. One preferred member used in the present invention, TIMP-1, is a secreted protein that can be detected on the cell surface through its interaction with surface proteins (Bode & Maskos, 2003).

The term "fusion construct", "TIMP construct", or "TIMP fusion construct" as used herein refer to both the nucleic acid molecule and the amino acid molecule encoded thereby.

The invention specifically relates to nucleic acids containing a nucleotide sequence including the sequence defined by SEQ ID NOS:1-3, or a homolog thereof, or unique fragments thereof. In the present invention, the sequence of a nucleic acid molecule that encodes the resulting protein is considered homologous to a second nucleic acid molecule if the nucleotide sequence of the first nucleic acid molecule is at least about 70% homologous, preferably at least about 80% homologous, and more preferably at least about 90% homologous to the sequence of the second nucleic acid molecule. Homology between two nucleic acid sequences may be readily determined using the known BLASTN algorithm (Altschul, *et al.,* 1990) with default settings. As a further example, another known test for ascertaining the homology of two nucleic acid sequences is whether they hybridize under normal hybridization conditions, preferably under stringent hybridization conditions.

Given the nucleic acid sequence disclosed herein, the skilled person can readily design nucleic acid structures having particular functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such *as in situ* hybridization, can be constructed. Numerous methods for labeling such probes with radioisotopes, fluorescent tags, enzymes, and binding moieties (e.g., biotin) are known, thus the probes of the invention can be readily adapted for easy detectability.

Oligonucleotides can also be designed and manufactured for other purposes. For example, the invention enables the design of antisense oligonucleotides, and triplex-forming oligonucleotides for use in the study of structure/function relationships. Homologous recombination can be implemented by adaptation of the presently described nucleic acid for use as a targeting means.

The protein encoded by the nucleic acid of the present invention further includes functional homologs. A protein is considered a functional homolog of another protein for a specific function, as described below, if the homolog has the same function as the other protein. The homolog can be, for example, a fragment of the protein, or a substitution, addition, or deletion mutant of the protein.

Determination of whether two amino acid sequences are substantially homologous is, for the purpose of the present invention, based on FASTA searches according to Pearson & Lipman (1988). For example, the amino acid sequence of a first protein is considered homologous to that of a second protein if the amino acid sequence of the first protein has at least about 70% amino acid sequence identity, preferably at least about 80% identity, and more preferably at least about 95% identity, with the sequence of the second protein.

The possibility of substituting one amino acid in a sequence with an equivalent amino acid is well-known. Groups of amino acids known to be equivalent include:
(a) Ala(A), Ser(S), Thr(T), Pro(P), Gly(G);
(b) Asn(N), Asp(D), Glu(E), Gln(Q);
(c) His(H), Arg(R), Lys(K);
(d) Met(M), Leu(L), Ile(I), Val(V); and
(e) Phe(F), Tyr(Y), Trp(W).

Substitutions, additions, and/or deletions in the amino acid sequences can be made as long as the protein encoded by the nucleic acid of the invention continues to satisfy the functional criteria described herein. An amino acid sequence that is substantially the same as another sequence, but that differs from the other sequence by means of one or more substitutions, additions, and/or deletions, is considered to be an equivalent sequence.

Preferably, less than 20%, more preferably less than 10%, and still more preferably less than 5%, of the number of amino acid residues in a sequence are substituted for, added to, or deleted from the protein encoded by the nucleic acid of the invention.

With the term "MMP" as used herein is meant a matrix metalloproteinase that belongs to the MMP superfamily as represented by at least 26 extracellular matrix-degrading metalloendopeptidases that are acting during tissue development and differentiation, cellular infiltration, wound healing, and as moderators of the immune response.

With the term "GPI" as used herein is meant glycoinositol phospholipids, in particular, glycosylphosphatidlyinositol as described in Medof *et al.,* 1996. These phospholipid-like anchors have a common structure for membrane attachment irrespective of protein function. GPI anchoring units are composed of a linear glycan containing a phosphoethanolamine, three mannose residues, and a non-acetylated glucosamine linked to an inositol phospholipid. The GPI sequence contains the signals that direct GPI anchoring.

A "TIMP-GPI" fusion construct as used herein relates to TIMP that is fused directly to a GPI-linkage sequence. The TIMP-GPI fusion construct is designed by substituting the 3'-mRNA or cDNA end sequence of naturally GPI-anchored proteins (i.e., a sequence that contains the signals that direct GPI anchoring) for the endogenous TIMP 3'-mRNA or cDNA end sequence.

The term "regeneration" generally refers to restoring the integrity of traumatized or otherwise injured tissue. This term can include the processes of wound healing, tissue repair, and other types of restorative activities occurring at the location where a physiological insult and ensuing tissue damage has occurred.

### DETAILED DESCRIPTION OF THE INVENTION

### The TIMP Family and Protein Engineering of Cellular Surfaces

Tissue inhibitors of metalloproteinases (TIMPs) are known as the major cellular inhibitors of the matrix metalloproteinase (MMP) sub-family, exhibiting varying degrees of efficacy against different MMP members, as well as different tissue expression patterns and modes of regulation. The TIMPs typically modulate the activity of soluble, matrix bound and cell associated MMPs. All four mammalian TIMPs have many broad similarities, but exhibit distinctive structural features, biochemical properties and expression patterns, which suggests that each TIMP has a particular in vivo function.

The TIMP-1 protein is the most widely expressed and studied member of the TIMP family. Other members of the TIMP family include TIMP-2, TIMP-3 and TIMP-4. TIMP proteins not only share common structural features, including a series of conserved cysteine residues that form disulfide bonds essential for the native protein conformation (Brew *et al.,* 2000), but they also have widely overlapping biological activities. The conserved N-terminal region of the TIMP proteins is necessary for functional inhibitory activities, while the divergent C-terminal regions are thought to modulate the selectivity of inhibition and binding efficiency of agents to the MMPs (Maskos & Bode, 2003). However, apart from their ability to act as MMP inhibitors, the various TIMP family members may also exhibit additional biological activities, including the regulation of proliferation and apoptosis in addition to the modulation of angiogenic and inflammatory responses.

TIMP-1 has been found to inhibit most MMPs (except MMP-2 and -14), and preferentially inhibits MMP-8. TIMP-1 is produced and secreted in soluble form by a variety of cell types and is widely distributed throughout the body. It is an extensively glycosylated protein with a molecular mass of 28.5 kDa. TIMP-1 inhibits the active forms of MMPs, and complexes with the proform of MMP9. Like MMP9, TIMP-1 expression is sensitive to many factors. Increased synthesis of TIMP-1 is caused by a wide variety of reagents that include: TGF beta, EGF, PDGF, FGFb, PMA, alltransretinoic acid (RA), IL1 and IL11.

TIMP-2 is a 21 kDa glycoprotein that is expressed by a variety of cell types. It forms a noncovalent, stoichiometric complex with both latent and active MMPs. TIMP-2 shows a preference for inhibition of MMP-2.

TIMP-3 is typically bound to the ECM and inhibits the activity of MMP-1, -2, -3, -9, and 13. TIMP-3 shows 30% amino acid homology with TIMP-1 and 38% homology with TIMP-2. TIMP-3 has been shown to promote the detachment of transformed cells from ECM and to accelerate morphological changes associated with cell transformation.

Due to its high-affinity binding to the ECM, TIMP-3 is unique among the TIMPs. TIMP-3 has been shown to promote the detachment of transformed cells from the ECM and to accelerate the morphological changes associated with cell transformation. TIMP-3 contains a glucosaminoglycan (GAG) binding domain comprising six amino acids (Lys30, Lys26, Lys22, Lys42, Arg20, Lys45) that are thought to be responsible for an association with the cell surface. TIMP-3 is the only TIMP that normally inhibits TACE (TNF-α-converting enzyme), another metalloprotease that releases soluble TNF and is responsible for the processing of the IL-6 receptor to thus play a central part in the wound healing process.

TIMP-4 inhibits all known MMPs, and preferentially inhibits MMP-2 and -7. TIMP4 shows 37 % amino acid identity with TIMP1 and 51 % homology with TIMP2 and TIMP3. TIMP4 is secreted extracellularly, predominantly in heart and brain tissue and appears to function in a tissue specific fashion with respect to extracellular matrix (ECM) homeostasis.

Protein engineering of cell surfaces is a potentially powerful technology through which the surface protein composition of cells can be manipulated without gene transfer. By substituting the mRNA derived cDNA sequence from a GPI-linked protein that contains the GPI signal domain for the carboxyl terminal region of a protein of interest, it is possible to generate a fusion construct that encodes a GPI-linked protein.

This approach offers multiple advantages over more traditional gene transfer approaches. For example, the method is applicable to cells that are difficult or impossible to transfect (e.g. primary microvascular endothelium, primary target cells, etc). The amount of protein added to the cell surface can be controlled and quantitated (by FACS or immunofluorescence). In addition, multiple GPI-anchored proteins can be sequentially or concurrently inserted into the same cells. Through molecular engineering it is possible to express an additional epitope tag that assists in protein purification as well as monitoring of reagent during experiments. The agent can be injected directly into the tumor or peritumorial area and the effect of selective leukocyte recruitment on tumor growth or FAS-induced apoptosis determined.

As used herein, the terms "isolated and/or purified" refer to the in vitro isolation of a DNA or polypeptide molecule from its natural cellular environment, and from association with other components of the cell, such as nucleic acid or polypeptides, so that it can be sequenced, replicated, and/or expressed. For example, "isolated GPI linking sequence" is RNA or DNA containing greater than 9, preferably 36, and more preferably 45 or more, sequential nucleotide bases that encode at least a portion of a linking sequence, or a variant thereof, or a RNA or DNA complementary thereto, that is complementary or hybridizes, respectively, to RNA or DNA encoding the linking sequence and remains stably bound under stringent conditions, as defined by methods well known in the art. Thus, the RNA or DNA is "isolated" in that it is free from at least one contaminating nucleic acid with which it is normally associated in the natural source of the RNA or DNA and is preferably substantially free of any other mammalian RNA or DNA.

As used herein, the term "recombinant nucleic acid" e.g., "recombinant DNA sequence" refers to a nucleic acid, e.g., to DNA, that has been derived or isolated from any appropriate tissue source, that may be subsequently chemically altered in vitro, so that its sequence is not naturally occurring, or corresponds to naturally occurring sequences that are not positioned as they would be positioned in a genome which has not been transformed with exogenous DNA. An example of DNA "derived" from a source, would be a DNA sequence that is identified as a useful fragment within a given organism, and which is then chemically synthesized in essentially pure form. An example of such DNA "isolated" from a source would be a useful DNA sequence that is excised or removed from said source by chemical means, e.g., by the use of restriction endonucleases, so that it can be further manipulated, e.g., amplified, for use in the invention, by the known methodology of genetic engineering.

Unlike conventional polypeptide anchors, which have different transmembrane sequences and connect to specific cytoplasmic extensions, these phospholipid-like anchors use a common structure as a general mechanism for membrane attachment irrespective of protein function. GPI anchoring units are composed of a linear glycan containing a phosphoethanolamine, three mannose residues, and a non-acetylated glucosamine linked to an inositol phospholipid. They are prefabricated in the endoplasmic reticulum (ER) and are added to primary translation products at the time of their translocation across the ER membrane. The GPI-modified products then are glycosylated in the ER and Golgi, and subsequently transported to the cell surface.

Preferred GPI-linking sequences that can be used in the present invention are derived from GPI-anchors that are isolated from, for example, enzymes such as alkaline phosphatase, acetylcholinesterase, 5' nucleotidase (CO73); adhesion molecules such as lymphocyte function-associated antigen (LFA-3; CD58), neural cell adhesion molecule (NCAM); complement regulatory proteins such as decay accelerating factor (DAF or CD55), or others such as the Fey receptor type III B (Fc-y-RIII or CD16b), Thy-1 (CD90), Qa-2, Ly-6A, Membrane inhibitor of reactive lysis (MIRL or CD59). For the purpose of the present invention, the lymphocyte function-associated antigen (LFA-3) is preferred. The skilled person will recognize that also any other of the known GPI-anchors can be used for the practice of the present invention.

For the construction of TIMP-GPI, either the full-length sequence of TIMP can be used in the fusion construct or a functionally active portion thereof, which retains the activity of TIMP. Likewise also a portion of the GPI sequence can be used as long as the portion allows for the incorporation of the TIMP protein into the surface cell membrane of cancer cells.

In the following, a plurality of embodiments relating to the TIMP fusion constructs are described, whereby the constructs were produced and provided for treatment of cancer and as agents in the field of regenerative medicine. In a first embodiment, the TIMP molecule is selected from the group consisting of TIMP-1, TIMP-2, TIMP-3 and TIMP-4, and is preferably fused to a GPI sequence.

In another preferred embodiment, the GPI sequence is 36 amino acids in length.

In a further embodiment, the TIMP protein that is selected from the group consisting of TIMP-1, TIMP-2, TIMP-3 and TIMP-4 and fused to the GPI sequence, or fused to a mucin domain or fractalkine domain followed by the GPI sequence, is truncated at the C-terminal. In a preferred embodiment, said TIMP molecule is truncated to the first 50, 50-100 or 50-152 N-terminal amino acid residues. More preferably, the TIMP molecule is truncated to the first 152 N-terminal amino acid residues and is the TIMP-1 molecule. The term "truncated" refers to a TIMP nucleic acid or amino acid sequence that contains less that the full number of nucleic acid bases or amino acid residues found in a native TIMP nucleic acid sequence or protein or to a nucleic acid or amino acid sequence has been deleted of non-desired sequences.

In yet a further embodiment, the TIMP fusion construct is defined by a sequence selected from the group consisting of SEQ ID NOs: 1, 2, and 3.

The obtained construct can then be expressed in any suitable cell line or host cell to obtain the functional TIMP polypeptide or protein. For this purpose, any of the suitable known vectors or plasmids can be used to express the GPI-anchored TIMP proteins of the present invention. As described in more detail below, target cancer cells treated with TIMP-GPI protein (and as control with rhTIMP-1 protein) were recognized by the protein constructs and, as consequence, killed due to FAS-mediated apoptosis.

In a preferred embodiment, and by way of example, one vector used for expression of the fusion constructs of the present invention contains the promoter for human elongation factor 1 alpha followed by a multiple cloning site and an internal ribosomal binding site which allows bicistronic expression of the construct and dihydrofolate reductase (DHFR) used as a selection marker (Mack, et al., P.N.A.S. USA 92:7021, 1995). The 3' end (carboxyl terminal) of the TIMP protein may be fused directly to a GPI-linkage sequence (e.g. derived from lymphocyte function-associated antigen-3 (LFA-3)). The resultant fusion constructs are transfected into dihydrofolate reductase (DHFR)-deficient Chinese hamster ovary (CHO) cells and the selection is performed as described (Mack, et al., P.N.A.S. USA 92:7021-7025, 1995). In a preferred embodiment, the transfectants can be exposed to methotrexate to increase the expression rate by gene amplification.

Although the use of TIMP-1 (Bode & Maskos, 2003) for the preparation of GPI-anchored TIMP protein is preferred in the present invention, the skilled person will recognize that also other TIMP proteins may be used for the practice of the present invention. Further examples of human TIMPs that are useful are TIMP-2, TIMP-3, and TIMP-4 (Mannello F, *et al.,* 2001). For example, in some embodiments, the sequence of TIMP-1 derived from an animal such as dog, cat, mouse, rabbit, cow or sheep, and bird may be used for the construction of a TIMP-GPI fusion construct of the present invention.

### The TIMP fusion Constructs for Use in Regenerative Medicine

Furthermore, the fusion constructs of the present invention are suitable for use in regenerative medicine, particularly in the area of wound healing. As described above, TIMP proteins that are fused to GPI or to mucin-GPI are efficiently incorporated into the cellular surface membrane, where they focus functional domains on those cell surfaces independently of protein-protein interactions. The TIMP fusion constructs of the present invention are typically quite stable and display amplified and novel bioactivities.

As described above, both MMPs and TACE play a crucial role in the process of wound healing. Increased MMP levels are associated with various wound healing disorders, *inter alia* chronic wound occurrence. Because the TIMPs are natural inhibitors of MMPs, the fusion constructs of the present invention can also be employed as effective therapeutic agents for accelerating the process of wound healing, for example, in regenerative medicine.

It was found in the present invention that wound healing increases the proliferation and migration of dermal keratinocytes.

A typical wound healing response is characterized by the movement of specialized cells into the wound site. Platelets and inflammatory cells are generally the first cells to arrive at the place of injury and these molecules provide important functions and chemical signals, including cytokines that are necessary for the influx of connective tissue cells and other healing factors. The term "wound" means a disruption of normal physiological structure and function. Thus, the wound healing process refers to the complex and dynamic sequence of events ultimately resulting in the restoration of physiological continuity and function.

Thus, the present invention offers effective regenerative agents and methods for accelerating the process of wound healing and/or to treat dysfunctions commonly associated with wound healing. Specifically, the fusion constructs of the present invention can be utilized to effectively control, alter, inhibit or even prevent these undesirable processes. The fusion constructs of the present invention can be formulated as a pharmaceutical and administered at the site of injury. In one embodiment, the site of injury is created by surgery, a burn, an injection, a bite, a vaccination, a trauma, surgery, or infection. In another embodiment, the fusion construct used for the preparation of the medicament to be applied to the site of injury is selected from the group consisting of TIMP-1-GPI, TIMP-2-GPI, TIMP-3-GPI, TIMP-4-GPI, or mutants thereof.

### Formulation of the TIMP constructs and modes of administration

Pharmaceutical compositions based on the TIMP constructs of the present invention may be formulated in a conventional manner using one or more physiologically acceptable carriers or excipients. Techniques and formulations generally may be found in Remrnington's Pharmaceutical Sciences, Meade Publishing Co., Easton, Pa*.* For the purposes of injection, the compounds of the invention can be formulated in a liquid solution, preferably in a physiologically compatible buffer such as Hank's solution or Ringer's solution. In addition, the compounds may be formulated in solid form and redissolved, or suspended immediately prior to use. Lyophilized forms are also suitable.

In addition to these formulations, the compounds may also be formulated as a depot preparation. These long acting depot formulations may be administered by implantation (for example, subcutaneously or intramuscularly) or by injection. Thus, the compounds may be formulated with suitable polymeric or hydrophobic materials (for example, as an emulsion in an acceptable oil) or as an ion exchange resin, or as a sparingly soluble derivative, such as a sparingly soluble salt. Other suitable delivery systems include microspheres, which offer the possibility of a local and noninvasive delivery of drugs over an extended period of time. This particular technology utilizes microspheres having a precapillary size that can be injected via a coronary catheter into any selected part of a tissue, e.g. the heart or other organs, without causing a resulting inflammation or ischemia. The administered therapeutic is slowly released from these microspheres and readily taken up by cells present in the surrounding tissue (e.g. injured or cancerous cells).

For topical administration, the oligomers of the invention can be formulated into ointments, salves, gels, or creams generally known in the art. A wash solution containing the oligomer can be used locally to treat an injury or inflammation or to generally accelerate the healing process.

The TIMP constructs of the present invention may be combined when preparing a medicament, so that the resulting medicament comprises more than one, preferably two, and even more preferably three different TIMP constructs. With this approach, the amplified and novel bioactivities of the different members of the TIMP family may be preferentially combined and targeted to the cell surface, which can lead to a synergistic effect. For example, the TIMP-1 fusion constructs inhibits most MMPs, except MMP-2 and MMP14. Therefore, any of the TIMP-1 constructs may be combined with any of the TIMP-2 or TIMP-4 constructs which both preferentially inhibit MMP-2. Therefore, by means of this combination, a more complete inhibition of the MMP family can be achieved.

In one embodiment, the formulations of the present invention therefore comprise a TIMP-1 construct, or a TIMP-2 and/or a TIMP-4 construct. In a preferred embodiment, the formulation comprises a TIMP-1 construct selected from the group consisting of a truncated TIMP-1-GPI as encoded by SEQ ID NO:1, and a TIMP-2 and/or a TIMP-4 construct. Preferably, the TIMP-2 construct is encoded by SEQ ID NO:3.

In a further embodiment, the formulation comprises a TIMP-3 construct of the present invention, preferably that encoded by SEQ ID NO:3, which inhibits TACE together with at least one of the TIMP constructs selected from the group consisting of a truncated TIMP-1-GPI as encoded by SEQ ID NO:1, a TIMP-2 and TIMP-4 construct. Preferably, the TIMP-1 and TIMP-2 constructs are encoded by SEQ ID NOs: 1, 2, and 3 respectively.

### BRIEF DESCRIPTION OF THE FIGURES

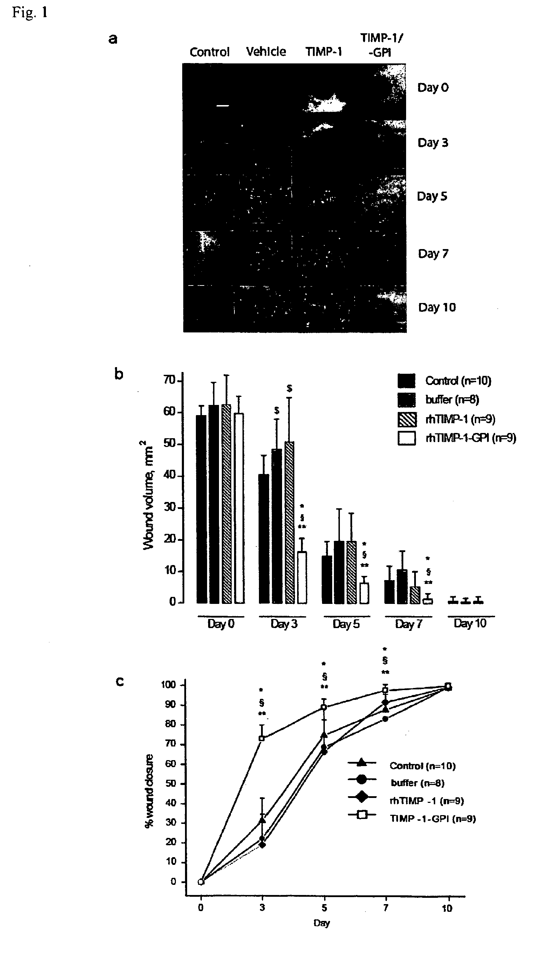

**Figure 1****. Effect of TIMP-1-GPI protein on wound healing.**
   Representative images (a) show wound healing in mice treated with rhTIMP-1 buffer, rhTIMP-1, or TIMP-1-GPI. Wounds were photographed at the times indicated, from days 0-10. Scale bar = 4 mm. The wound volume (b) and the wound-closure rate (c) under TIMP-1-GPI treatment relative to that achieved by rhTIMP-1 buffer (§p < 0.05) or rhTIMP-1 (**p < 0.05) application. *p < 0.05, #p < 0.05 (both versus Control).

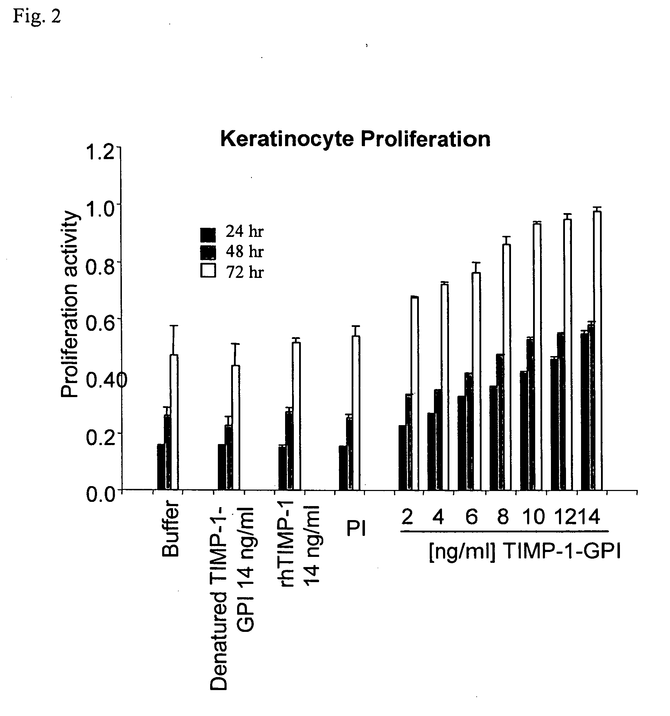

**Figure 2****. Treatment with GPI-anchored TIMP-1 stimulates keratinocyte cell proliferation**
   To assess the effect of TIMP-1 surface engineering on proliferation of primary human keratinocytes, MTT assays were performed. TIMP-1-GPI treated keratinocytes show a dose dependant increase in proliferation (at 24, 48 and 72 hrs, reflected in the three columns). Absorbance was then measured at 550nm using GENios plus TECAN ELISA reader. For each experiment, at least six wells were analysed per experimental condition and time point. PI (phosphatidylinositol (GPI anchor).

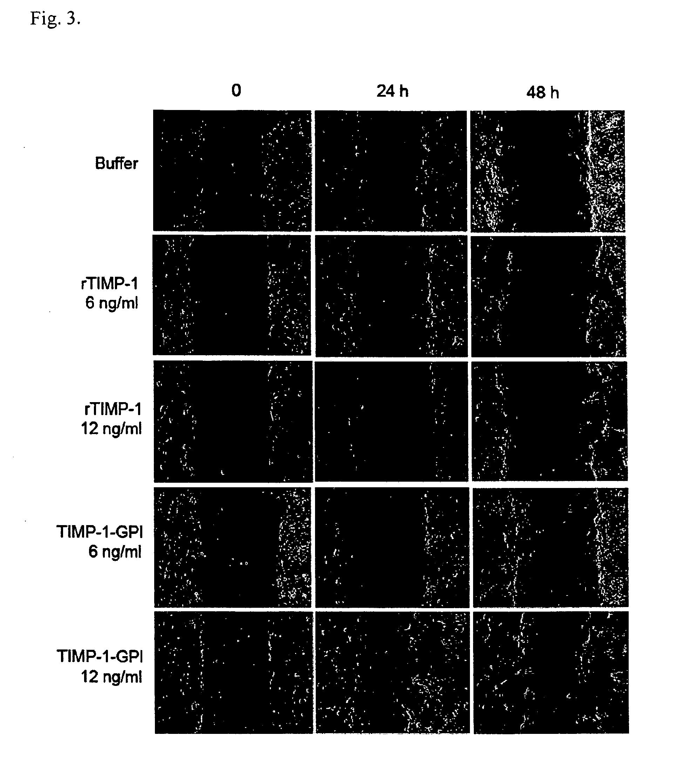

**Figure 3****. In vitro wound healing assay shows increased wound closure following application of TIMP-1-GPI.**
   The effect of TIMP-1-GPI on a monolayer of primary human keratinoytes as observed in light microscopy is shown.
   An in vitro wound healing assay using a commercially available system (Ibidi, Martinsried) was used to test the effect of TIMP-1-GPI treatment on the migration and proliferation of human dermal keratinocytes. In this assay, the cells were cultured on plastic in a chamber. When the chamber was removed, this lead to the generation of a uniform wound in the monolayer of keratinocytes. The "repair" of this wound was then observed over 48 hours. The results show that treatment with vehicle or control TIMP-1 does not lead to improved wound closure. By contrast, TIMP-1-GPI at 12 ng/ml shows pronounced closure by 24 hours after treatment. The higher the concentration of TIMP-1-GPI the faster the "wound" (the "wound" in clearly visible on day 0) is closed.

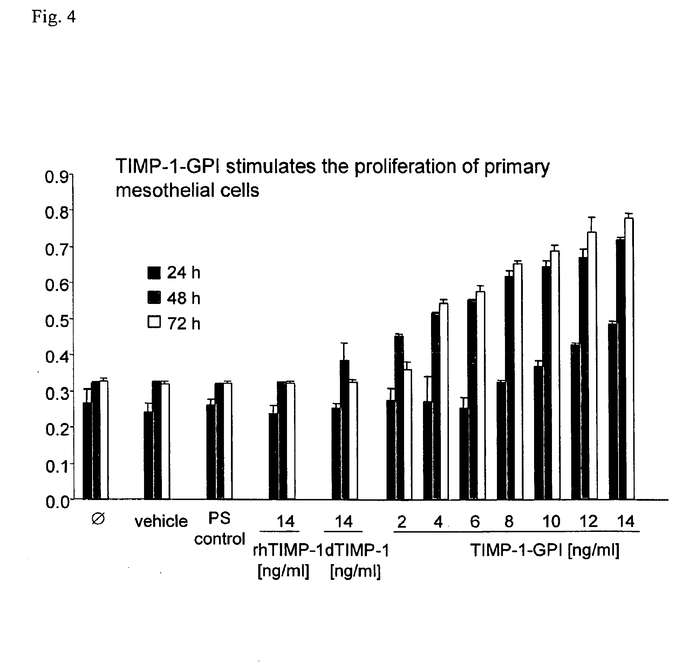

**Figure 4****. TIMP-1-GPI stimulates the proliferation of primary mesothelial cells.**
   The effect of TIMP-1-GPI on the proliferation of cultured primary mesothelial cells is shown. The vehicle is 1xPBS 0.05% TritonX100.
   To assess the effect of TIMP-1 surface engineering on proliferation of primary human mesothelial cells, MTT assays were performed. TIMP-1-GPI treated mesothelial cells show a dose dependant increase in proliferation (at 24, 48 and 72 hrs). Absorbance was then measured at 550nm using GENios plus TECAN ELISA reader. For each experiment, at least six wells were analysed per experimental condition and time point.

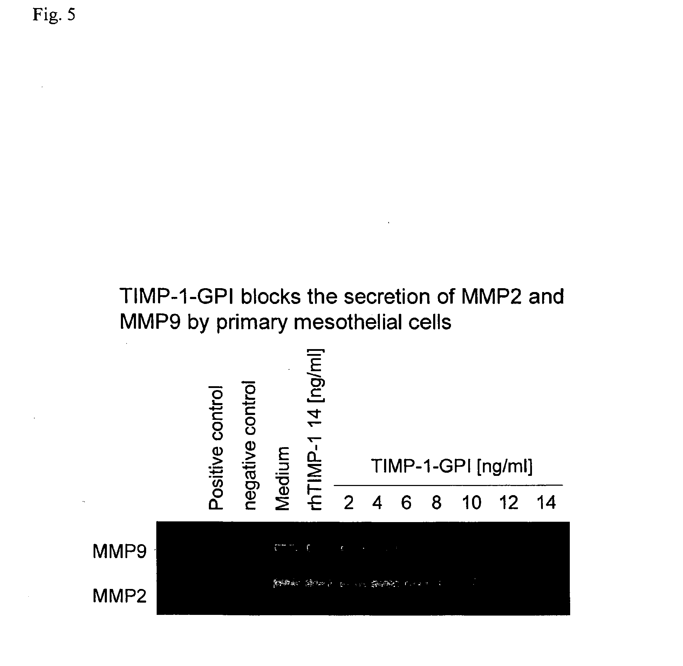

**Figure 5****. Zymograpy. Influence of TIMP-1-GPI on the secretion of MMP2 and MMP9.**
   Primary human fibroblast cells were cultured in 24-well plate (5_104 cells/well). The medium was exchanged for 24 h with serum-free medium containing either rhTIMP-1 or increasing amounts of TIMP-1- GPI and incubated for 24, 48 and 72 h. Cell supernatants were analysed by gelatin zymography using 10% SDS-polyacrylamide gels (Invitrogen, Groningen, the Netherlands; No. EC61755BOX) as described (Djafarzadeh et al., 2004). Recombinant MMP-9 enzyme (Amersham Biosciences, Uppsala, Sweden; No. RPN2634) was used as positive control.

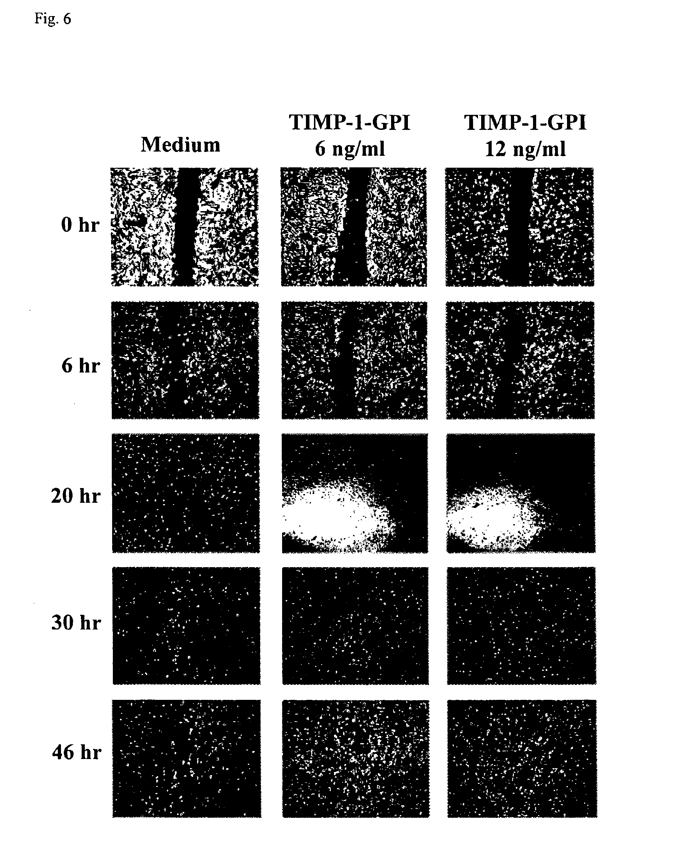

**Figure 6****. Mesothelial cell mobilization and proliferation is increased by TIMP-1-GPI.**
   The effect of TIMP-1-GPI on a monolayer of primary human mesothelial cells as observed in light microscopy is shown.
   An in vitro wound healing assay using a commercially available system (Ibidi, Martinsried) was used to test the effect of TIMP-1-GPI treatment on the migration and proliferation of primary human mesothelial cells. In this assay, the cells were cultured as a monolayer on plastic using an Ibidi wound chamber. When the chamber was removed, this lead to the generation of a uniform wound in the monolayer of mesothelial cells. The "repair" of this wound was then observed over 48 hours. The results show that treatment with vehicle or control TIMP-1 does not lead to improved wound closure. By contrast, TIMP-1-GPI at 14 ng/ml shows pronounced closure by 24 hours after treatment. The higher the concentration of TIMP-1-GPI the faster the "wound" (the "wound" in clearly visible at 0 hr) is closed.

### EXAMPLES

### Example 1

### Effect of TIMP-1-GPI on wound healing in vivo

To determine the effect of TIMP-GPI on wound healing in vivo a murine excisional wound model was employed. C57BL/6 mice were anesthetized with ketavet (100 mg/kg mouse body weight) and xylazin (5 mg/kg mouse body weight) followed premedication with atropine sulfate.

After the dorsal surface of each mouse had been disinfected with an alcohol swab, a full-thickness excisional wound (0.8 cm in diameter) was created on the dorso-medial back of each animal, using a standard skin biopsy punch (Stiefel® Laboratorium GmbH, Germany). Immediately after surgery, as well as on days 3, 5, and 7 thereafter, rhTIMP-1 buffer, or rhTIMP-1, or TIMP-1-GPI in an amount that was sufficient to cover the wound, generally between 50 to 200 µl, were applied on the top of created wound.

The measurement of wound size was performed on days 0, 3, 5, 7, and 10. The wound area (mm²) was calculated, using the formula of an ellipse, the wound-closure rate was expressed as a ratio of the wounded area at each time point divided by the area of the original wound at time 0. This is a formula that can be used throughout the application to determine the wound-closure rate. On postoperative day 14, the animals were euthanized. The results are displayed in figure 1.

Wound area and wound-closure rate are expressed as the mean ± SD. The data were analyzed using the paired Student's t-test. Analyses were computed using the statistical package in Microcal Origin version 6.0 (Microcal Software, MA, USA) with p < 0.05 considered to be significant.

The results demonstrate a significantly accelerated wound closure rate by day three after initiation of treatment TIMP-1-GPI in a mouse model. This was evidenced by measurement of wound volume as well as the percent of wound closure (Figure 1). In *in vitro* assays, application of GPI-anchored TIMP-1 was found to induce keratinocyte proliferation and mobilization leading to a more rapid wound healing (Figure 2). Complete closure is seen with 12 ng/ml at 48 hrs.

This is contrary to what was previously reported for dermal fibroblasts where treatment suppressed proliferation, migration and activation (see WO2007039109).

### Example 2

### Dermal keratinocyte mobilization and proliferation is increased by TIMP-1-GPI

In dermal wounds, following the inflammatory response, keratinocyte mobilization is required as the cells migrate into the wound to initiate closure. An in vitro wound healing model was used to study the effect of TIMP-1-GPI on keratinocyte activity. A monolayer of primary human keratinocytes was cultured in an insert provided by Ibidi (Munich, Germany). Removal of the insert yielded a uniform "wound" to the monolayer. The closure of the wound was then determined in the context of TIMP treatment over three days (Figure 3).

Treatment with increasing levels of TIMP-1-GPI lead to rapid closure of the wound. Control rhTIMP-1, denatured TIMP-1-GPI or phosphatidyl inositol had no significant effect on the wound closure. In parallel, treatment was found to increase in a dose dependent way, the proliferation of keratinocytes.

### Example 3

### TIMP-1-GPI treatment increased the proliferation and migration of primary human mesothelial cells suggesting it will show efficacy in mesothelial repair

The mesothelium is a flat epithelial lining of the peritoneal, pleural, and pericardial cavities. It provides a nonadhesive, slippery surface and gates the traffic of molecules and cells between the circulation and these body compartments. Given its strategic location, it is likely that the mesothelial lining participates actively in regulating leucocyte traffic between the circulation, the peritoneal fluid, and the walls of the peritoneal cavity.

The peritoneal mesothelium has been implicated in the long-term development of ultrafiltration failure in peritoneal dialysis patients. The presence of supra-physiological glucose concentrations, acidity, and glucose degradation products in peritoneal dialysis fluids contribute to the fibrosis of the peritoneal mesothelium, either by epithelial-mesenchymal transition or increased proliferation of existing fibroblasts. A fibrosed peritoneum results in the increased passage of solutes across the peritoneum and ultrafiltration failure. We have previously shown that TIMP-1-GPI treatment can reduce the activation, proliferation and migration of fibroblasts. Similar to what was shown above for dermal keratinocytes, and contrary to what was shown for fibroblasts, treatment of primary human mesothelial cells with TIMP-1-GPI was found to stimulate their proliferation suggesting that TIMP-1-GPI may help repair damaged mesothelium (Figure 4).

Treatment of mesothelial cells with TIMP-1-GPI lead to a dose dependent stimulation of proliferation. This is contrary to what was found for activated fibroblasts. Mesothelial cells (30 x 10³/100 µl medium) were cultured in 96-well micro-titer plates for 24 h under standard conditions to yield firmly attached and stably growing cells. After discarding supernatants, 50µl of medium containing TIMP-1-GPI, buffer, or rhTIMP-1 was added to cells and incubated for 24 to 72 h. Then 50µl of a 1 mg/ml solution of (3,5- Dimethylthiazol-2-yl] - 2,5 - diphenyl-tetrazolium bromide) MTT (SIGMA-ALDRICH, Taufkirchen, Germany No. M2128) was added. After 3 h incubation at 37°C, formazan crystals were dissolved by addition 100µl isopropanol and 0.04 N HCl. Absorbance was then measured at 590 nm using GENios plus TECAN ELISA reader. For each experiment at least 6-wells were analyzed per experimental condition and time point (Figure 4).

### Example 4

### Zymography

Treatment of mesothelial cells with increasing amounts of TIMP-1-GPi lead to a dose dependent blockade of MMP2 and MMP9 secretion into the growth medium.

Mesothelial cells were cultured in 24 wells plate (5 x 10⁴ cells/well). The medium was exchanged for 24 h with serum-free medium containing either rhTIMP-1, or other controls and increasing amounts of TIMP-1-GPI and incubated for 24 h, 48 h and 72 h. Cell supernatants were analyzed by gelatin zymography using 10% SDS-polyacrylamide gels (Invitrogen, Groningen, Netherlands, No. EC61755BOX) as described (Djafarzadeh, 2004). Rcombinant MMP-9 enzyme (Amersham Biosciences, Uppsala, Sweden, No. RPN2634) was used as positive control (Figure 5).

### Example 5

### In vitro wound repair

In repair of the mesothelium, following the inflammatory response mesothelial migration is required. An in vitro wound healing model was used to study the effect of TIMP-1-GPI on mesothelial activity. A monolayer of primary human mesothelial cells was cultured in an insert provided by Ibidi (Munich, Germany). Removal of the insert yielded a uniform "wound" to the monolayer. The closure of the wound was then determined in the context of TIMP treatment over 46 hrs.

Treatment with increasing levels of TIMP-1-GPI lead to rapid closure of the wound (Figure 6).

### Materials and Methods

### Cellular reagents and tissue culture

Primary human keratinocytes and primary human dermal fibroblast cells were obtained from PromoCell (Heidelberg, Germany No. C-12300) were cultured as per the companies directions.

### Purification of TIMP-1-GPI protein

The TIMP-1-GPI protein was produced and purified as previously described (Djafarzadeh*, et al.* 2004). Briefly, human TIMP-1 was cloned from cDNA using hTIMP-1 specific primers, fused without a translation stop codon to the GPI-signal sequence cloned from LFA-3 (Kirby *et al.,* 1995; Medof *et al.,* 1996) and subcloned into pEF-DHFR and stably introduced into DHFR deficient Chinese hamster ovary (CHO) cells and selected as described (Mack *et al.,* 1995). TIMP-1-GPI-fusion protein was purified from the CHO cells by Triton X-100 detergent extraction followed by column purification using DEAE, heparin sepharose and size exclusion (Djafarzadeh *et al.,* 2004).

### TIMP-1 ELISA

A human TIMP-1 specific ELISA using the protocol applied according to the manufacture's directions (MAB970, R&D Systems) was used to monitor levels of TIMP-1 in solution. The coating anti-human TIMP-1 mAB (MAB970), biotinylated anti-human TIMP-1 detection mAB (BAF970) and rhTIMP-1 protein (970-TM) were purchased from R&D Systems GmbH (Wiesbaden, Germany).

### GPI-anchor cleavage by phospholipase C

Cells (5-10 x 10⁶ cells/ml) were incubated with 200 or 700 ng of TIMP-1-GPI or rhTIMP-1 protein in serum-free medium for 1 h at 37°C by 5% CO2. The cells were washed three times with cold PBS and treated with 60 ng/ml phosphatidylinositol-specific phospholipase C (SIGMA-ALDRICH, Taufkirchen, Germany No. 661-9) in serum-free medium for 30 min at 37°C/5% CO2. Cells were washed three times, all supernatants were harvested.

### Proliferation

Primary keratinocytes and mesothelian cells (30 x 10³/100 µl medium) were cultured in 96-well micro-titer plates for 24 h under standard conditions to yield firmly attached and stably growing cells. After discarding supernatants, 50 µl of medium containing TIMP-1-GPI, buffer, or rhTIMP-1 was added to cells and incubated for 24 to 72 h. Then 50 µl of a 1 mg/ml solution of (3,5- Dimethylthiazol-2-yl]-2,5-diphenyl-tetrazolium bromide) MTT (SIGMA-ALDRICH, Taufkirchen, Germany No. M2128) was added. After 3-h incubation at 37°C, formazan crystals were dissolved by addition 100 µl isopropanol and 0.04 N HCl. Absorbance was then measured at 550 nm using GENios plus TEC AN ELISA reader. For each experiment at least 3 wells were analyzed per experimental condition and time point.

### Zymography

Mesothelian cells were cultured in 24 well plate (5 x 10⁴ cells/well). The medium was exchanged for 24 h with serum-free medium containing either rhTIMP-1 or increasing amounts of TIMP-1-GPI and incubated for 24 h, 48 h and 72 h. Cell supernatants were analyzed by gelatin zymography using 10% SDS-polyacrylamide gels (Invitrogen, Groningen, Netherlands, No. EC61755BOX) as described (Djafarzadeh *et al.,* 2004). Recombinant MMP-9 enzyme (Amersham Biosciences, Uppsala, Sweden, No. RPN2634) was used as positive control.

### Western Blot

Western blot was used for the detection of MMPs in serum free growth media. The anti-MMP antibodies used are described above (FACS analysis). Recombinant human MMP Western blotting standards were purchased from R&D Systems (Minneapolis, USA) and included; MMP-1 (WBC024), MMP-2 (WBC025), MMP-3 (WBC015), MMP-8 (WBC017), MMP-12 (WBC019) and MMP-13 (WBC020). Western blot was also used for the detection of BCL-2, BAX (see above for mAbs) and β-actin (Acris Hiddenhausen, Germany, No. ab8227). All proteins were detected using a commercial Western blot analysis kit, Chemiluminescent Immunodetection System (Invitrogen, Groningen, Netherlands).

### REFERENCES

Altschul, S.F., Gish, W., Miller, W., Myers, E.W. & Lipman, D.J. (1990) J. Mol. Biol. 215:403-410.
Ahonen, M., Poukkula, M., Baker, A.H., Kashiwagi, M., Nagase, H., Eriksson, J.E. & Kahari, V.M. (2003). Oncogene, 22, 2121-34.
Baker, A.H., George, S.J., Zaltsman, A.B., Murphy, G. & Newby, A.C. (1999). BrJCancer, 79, 1347-55.
Baker, A.H., Zaltsman, A.B., George, S.J. & Newby, A.C. (1998). JClin Invest, 101, 1478-87.
Barnstable, C.J., Jones, E.A. & Crumpton, M.J. (1978). BrMedBull, 34, 241-6.
Bigda, J., Beletsky, I., Brakebusch, C., Varfolomeev, Y., Engelmann, H., Holtmann, H. & Wallach, D. (1994). JExpMed, 180, 445-60.
Bloomston, M., Shafii, A., Zervos, E.E. & Rosemurgy, A.S. (2002). JSurgRes, 102, 39-44. Bode, W. & Maskos, K. (2003). BiolChem, 384, 863-72.
Bond, M., Murphy, G., Bennett, M.R., Newby, A.C. & Baker, A.H. (2002). JBiol Chem, 277, 13787-95.
Brand, K. (2002). Curr Gene Ther, 2, 255-71.
Brew, K., Dinakarpandian, D. & Nagase, H. (2000). Biochim Biophys Acta, 1477, 267-83.
Brown, O., Cowen, R.L., Preston, C.M., Castro, M.G. & Lowenstein, P.R. (2000). Gene Ther, 7, 1947-53.
Djafarzadeh, R., Mojaat, A., Vicente, A.B., von Luttichau, I. & Nelson, P.J. (2004). Biol Chem, 385, 655-63.
Dunn, G.P., Old, L.J. & Schreiber, R.D. (2004). Annu Rev Immunol, 22, 329-60.
Egeblad, M. & Werb, Z. (2002). Nat Rev Cancer, 2, 161-74.
Frost, P., Caliliw, R., Belldegrun, A. & Bonavida, B. (2003). IntJ Oncol, 22, 431-7.
Giard, D.J., Aaronson, S.A., Todaro, G.J., Arnstein, P., Kersey, J.H., Dosik, H. & Parks, W.P. (1973). JNatl CancerInst, 51, 1417-23.
Gong, J.H., Maki, G. & Klingemann, H.G. (1994). Leukemia, 8, 652-8.
Hemmerlein, B., Johanns, U., Halbfass, J., Bottcher, T., Heuser, M., Radzun, H.J. & Thelen, P. (2004). IntJ Oncol, 24, 1069-76.
Igney, F.H. & Krammer, P.H. (2002). Nat Rev Cancer, 2, 277-88.
Itoh, Y. & Nagase, H. (2002). Essays Biochem, 38, 21-36.
Johnson, J.P., Stade, B.G., Hupke, U., Holzmann, B. & Riethmuller, G. (1988). Immunobiology, 178, 275-84.
Kagi, D., Vignaux, F., Ledermann, B., Burki, K., Depraetere, V., Nagata, S., Hengartner, H. & Golstein, P. (1994). Science, 265, 528-30.
Kirby, A.C., Hill, V., Olsen, I. & Porter, S.R. (1995). Biochem Biophys Res Commun, 214, 200-5.
Klier, C.M., Nelson, E.L., Cohen, C.D., Horuk, R., Schlondorff, D. & Nelson, P.J. (2001). Biol Chem, 382, 1405-10.
Lang, R., Braun, M., Sounni, N.E., Noel, A., Frankenne, F., Foidart, J.M., Bode, W. & Maskos, K. (2004). JMolBiol, 336, 213-25.
Maskos K. and Bode W. (2003) Mol Biotechnol 25:241-266.
Mack, M., Riethmuller, G. & Kufer, P. (1995). Proc NatlAcadSci USA, 92, 7021-5.
Majid, M.A., Smith, V.A., Easty, D.L., Baker, A.H. & Newby, A.C. (2002). BrJ Ophthalmol, 86, 97-101.
Mannello F., Gazzanelli, G. (2001), Apoptosis, 6(6):479-82.
Medof, M.E., Nagarajan, S. & Tykocinski, M.L. (1996). FASeb J, 10, 574-86.
Milani, V., Frankenberger, B., Heinz, O., Brandl, A., Ruhland, S., Issels, R.D. & Noessner, E. (2005). Int Immunol*.*
Moniaux N., Adrianifahanana M., Brand R.E., Batra S.K. (2004), British Journal of Cancer, 91, 1633-1638
Nagase, H. & Woessner, J.F., Jr. (1999). JBiolChem, 274, 21491-4.
Nagata, S. (1999). Annu Rev Genet, 33, 29-55. Parham, P. & Brodsky,
F.M. (1981). Hum Immunol, 3, 277-99. Rigg, A.S. & Lemoine, N.R. (2001). Cancer Gene Ther, 8, 869-78.
Pearson WR & Lipman DJ. (1988). Proc Natl Acad Sci USA 85:2444-2448
Robertson, M.J., Cochran, K.J., Cameron, C., Le, J.M., Tantravahi, R. & Ritz, J. (1996). Exp Hematol, 24, 406-15.
Sayers, T.J., Brooks, A.D., Lee, J.K., Fenton, R.G., Komschlies, K.L., Wigginton, J.M., Winkler-Pickett, R. & Wiltrout, R.H. (1998). J Immunol, 161, 3957-65.
Schendel, D.J., Frankenberger, B., Jantzer, P., Cayeux, S., Noessner, E., Willimsky, G., Maget, B., Pohla, H. & Blankenstein, T. (2000). Gene Ther, 7, 2007-14.
Schendel, D.J., Gansbacher, B., Oberneder, R., Kriegmair, M., Hofstetter, A., Riethmuller, G. & Segurado, O.G. (1993). J Immunol, 151, 4209-20.
Schendel, D.J., Oberneder, R., Falk, C.S., Jantzer, P., Kressenstein, S., Maget, B., Hofstetter, A., Riethmuller, G. & Noessner, E. (1997). JMolMed, 75, 400-13.
Seki, N., Brooks, A.D., Carter, C.R., Back, T.C., Parsoneault, E.M., Smyth, M.J., Wiltrout, R.H. & Sayers, T.J. (2002). J Immunol, 168, 3484-92.
Singer A. and Clark R. (1999). N. Engl. J. Med., 341, 738-746.
Smith, M.R., Kung, H., Durum, S.K., Colburn, N.H. & Sun, Y. (1997). Cytokine, 9, 770-80.
Span, P.N., Lindberg, R.L., Manders, P., Tjan-Heijnen, V.C., Heuvel, J.J., Beex, L.V. &
Sweep, C.G. (2004). *JPathol,* 202, 395-402.
Trapani, J.A., Davis, J., Sutton, V.R. & Smyth, M.J. (2000). Curr Opin Immunol, 12, 323-9.
Vogelzang, N.J. & Stadler, W.M. (1998). Lancet, 352, 1691-6.
Zacchigna, S., Zentilin, L., Morini, M., Dell'Eva, R., Noonan, D.M., Albini, A. & Giacca, M. (2004). Cancer Gene Ther, 11, 73-80.

## Claims

1. Use of a fusion construct, comprising an amino acid sequence of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor, for the preparation of a medicament for accelerating wound healing.

2. The use of claim 1, wherein said TIMP is selected from the group consisting of TIMP-1, TIMP-2, TIMP-3, or TIMP-4.

3. The use of any one of claims 1-2, wherein said TIMP is human TIMP-1.

4. The use of any one of claims 1-3, wherein said fusion construct contains one or more GPI signal sequences to direct GPI-anchoring.

5. The use of any one of claims 1-4, wherein said GPI-anchor is derived from the lymphocyte function-associated antigen (LFA-3), or a portion thereof.

6. The use of any one of claims 1-5, wherein said fusion construct is to be administered at a concentration of 0.005 to 0.1 µg/ml, preferably 0.005 to 0.015 µg/ml.

7. The use according to any one of claims 1-7, wherein the pharmaceutical composition is used together with a detergent, sealant or carrier substance.

8. *An in vitro* method comprising adding an amino acid sequence of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor to primary keratinocytes or mesothelial cells in culture and measuring the proliferation and/or mobilization of said cells.

9. The method of claim 9, wherein said TIMP is selected from the group consisting of TIMP-1, TIMP-2, TIMP-3, or TIMP-4.

10. The method of any one of claims 9-10, wherein said TIMP is human TIMP-1.

11. The method of any one of claims 9-11, wherein said fusion construct contains one or more GPI signal sequences to direct GPI-anchoring.

12. The method of any one of claims 9-12, wherein said GPI-anchor is derived from the lymphocyte function-associated antigen (LFA-3), or a portion thereof.

13. The method of any one of claims 9-13, wherein said fusion construct is to be administered at a concentration of 0.005 to 0.1 µg/ml, preferably 0.005 to 0.015 µg/ml.

14. The method according to any one of claims 9-15, wherein the pharmaceutical composition is used together with a detergent, sealant or carrier substance.

15. A pharmaceutical composition comprising 0.005 to 0.1 µg/ml, preferably 0.005 to 0.015 µg/ml of a tissue inhibitor of metalloproteinases (TIMP) or a biologically active fragment thereof that inhibits active matrix metalloproteinases, wherein said TIMP or biologically active fragment thereof is linked to a glycosylphosphatidylinositol (GPI)-anchor.
